# EUROPÄISCHE PATENTSCHRIFT

(11) **EP 4 124 307 B1**
(45) Veröffentlichungstag und Bekanntmachung des Hinweises auf die Patenterteilung: **04.09.2024**
(21) Anmeldenummer: 21187775.8
(22) Anmeldetag: 26.07.2021
(51) Int. Cl.: A61B 17/3203, A61B 18/00, A61B 18/14, A61B 18/12

(54) **ELEKTROCHIRURGISCHES INSTRUMENT MIT BEWEGLICHER ELEKTRODE**
ELECTROSURGICAL INSTRUMENT WITH MOVABLE ELECTRODE
INSTRUMENT ÉLECTROCHIRURGICAL À ÉLECTRODE MOBILE

(43) Veröffentlichungstag der Anmeldung: 01.02.2023
(73) Patentinhaber: Erbe Elektromedizin GmbH, 72072 Tübingen (DE)
(72) Erfinder: Karwei, Dietmar, 72131 Ofterdingen (DE); Bader, Fabian, 72108 Rottenburg (DE)
(74) Vertreter: Rüger Abel Patentanwälte PartGmbB

(56) Entgegenhaltungen:
- EP-B1- 3 187 137
- WO-A1-2020/070113
- US-A1- 2020 352 643

## Beschreibung

Die Erfindung betrifft ein elektrochirurgisches Instrument, insbesondere ein Hybridinstrument, das sowohl eine Elektrode als auch eine Fluiddüse aufweist.

Aus der DE 36 16 616 A1 ist ein als Hochfrequenz-Schneidevorrichtung bezeichnetes Instrument bekannt, das aus einem Schlauch besteht, in dessen distales Ende eine Gewindebuchse eingesetzt ist. Die Stirnfläche dieser Gewindebuchse ist gegen die distale Stirnfläche des flexiblen Schlauchs in proximaler Richtung zurück versetzt. Die Gewindebuchse weist eine Durchgangsöffnung auf, die an ihrem distalen Ende verengt ist. Durch den verengten Bereich der Gewindebuchse erstreckt sich eine Elektrode, die in einer Fassung gehalten ist. Die Fassung sitzt in dem Durchgangskanal, erstreckt sich aber nicht in den verengten Bereich hinein.

In einer weiteren in dieser Druckschrift veranschaulichten Ausführungsform ist der Durchgangskanal konisch, wobei die Fassung ebenfalls konisch ausgebildet ist. Auch bei dieser Ausführungsform kann die Fassung nicht bis an das distale Ende des Instruments oder auch nur der Gewindebuchse bewegt werden.

Weiterer gattungsgemäßer Stand der Technik geht aus der US 2020/352643 A1, der EP 1 752 108 A1, der EP 2 156 801 A1, der EP 1 726 267 B1 und der US 6,193,717 B1 hervor.

Bei solchen Instrumenten ist die Elektrode häufig als hohles filigranes Röhrchen ausgebildet, das in einer Rückzugsposition innerhalb des Instruments steht und in einer Ausschubposition aus dem distalen Ende des Instruments heraus ragt. Die Elektrode ist dabei schlank, d.h. im Verhältnis zu ihrem Durchmesser lang und somit bei zunehmender Miniaturisierung solcher Instrumente bruchgefährdet.

Es ist Aufgabe der Erfindung, ein verbessertes Instrument anzugeben.

Diese Aufgabe wird mit dem elektrochirurgischen Instrument nach Anspruch 1 gelöst:

Das erfindungsgemäße Instrument weist ein distales Endstück mit einer distalen Endfläche und einem Durchgangskanal auf, wobei die distale Endfläche eine Mündungsöffnung umschließt, an der der Durchgangskanal mündet. In dem Durchgangskanal ist eine Fassung angeordnet, die zwischen einer Rückzugsposition und einer Ausschubposition linear in Axialrichtung bewegbar ist. Die Axialrichtung erstreckt sich von einem proximalen Abschnitt des Endstücks zu der Mündungsöffnung hin. Die Fassung schließt in Ausschubposition mit der Endfläche bündig ab oder ragt aus dieser heraus. Ein distaler Abschnitt der Fassung weist eine Sitzöffnung auf, in der die Elektrode angeordnet ist. Diese ragt mit einem Abschnitt aus der Sitzöffnung heraus.

Durch diese Gestaltung ist die Länge des aus der Fassung herausragenden Abschnitts der Elektrode minimiert, wodurch die Bruchgefahr der Elektrode gegenüber Gestaltungen verringert ist, bei denen die Fassung der Elektrode hinter der distalen Endfläche des Endstücks zurückbleibt, wie es im Stand der Technik der Fall ist. Bei der Erfindung wird die Länge des aus der Fassung herausragenden Abschnitts der Elektrode so kurz wie möglich gehalten, wodurch das Verhältnis zwischen der Länge dieses Abschnitts und seinen Durchmesser (Aspektverhältnis) vergrößert werden kann. Dies ist insbesondere bei dünnen Elektroden von Vorteil, die außerdem hohl ausgebildet sind, d.h. die ein Lumen aufweisen, mit dem Flüssigkeit durch die Elektrode geführt und an deren distalen Ende abgegeben werden kann. Solche Elektroden können beispielsweise aus Wolfram oder Hartmetall im Metal-Injection-Molding-Verfahren (MIM) oder in anderen geeigneten Verfahren hergestellt werden. Bei der erfindungsgemäßen Anordnung kann die Fassung als Wärmesenke für die Elektrode dienen, die sich im Einsatz stark erhitzen kann. Die Wärmekapazität der Fassung dient der Kühlung der Elektrode und vermindert somit schädliche erwärmungsbedingte Erscheinungen, wie beispielsweise Versprödung des Materials der Elektrode, Anhaftung von Gewebe, erzwungene Pausenzeiten zwischen aufeinanderfolgenden Aktivierungen und dergleichen. Mit der erfindungsgemäßen Gestaltung kann die Fassung die von der Elektrode ausgehende Wärme auf kurzem Wege aufnehmen.

Das erfindungsgemäße Konzept gestattet die Minimierung der Länge der Elektrode. Die Länge der Elektrode setzt sich aus der Länge des aus der Fassung herausragenden Abschnitts und der Länge des in der Fassung gehaltenen Abschnitts zusammen. Enthält die Elektrode einen Fluidkanal ist dessen Länge und somit dessen Strömungswiderstand gleichfalls minimiert. Damit wird der Fluiddruck gesenkt, der nötige ist, um am distalen Ende der Elektrode einen zum Unterspritzen von Gewebe geeigneten Fluidstrahl zu erzeugen. Entsprechend kann die zum Fördern des Fluids nötige Pumpenleistung gesenkt werden.

Das erfindungsgemäße Konzept ermöglicht eine vereinfachte Montage der Elektrode. Diese kann in die im distalen Endstück positionierte Fassung eingeschoben und dann in dieser fixiert werden. Zur Fixierung der Elektrode kann eine Laserschweißverbindung wischen der Elektrode und der Fassung genutzt werden. Die Laserschweißverbindung kann angebracht werden sobald die Fassung in Ausschubposition steht. Die Fügestelle zwischen der Fassung und der Elektrode liegt somit in der gleichen Ebene, wie die distale Endfläche des Endstücks oder sogar distal vor dieser und ist somit leicht zugänglich.

Bei einer bevorzugten Ausführungsform weist der in dem Endstück ausgebildete Durchgangskanal einen verengten distalen Abschnitt auf, der über eine Schulter an den sonstigen vorzugsweise zylindrisch ausgebildeten Durchgangskanal anschließt. Die Schulter bildet ein Anschlagmittel für die Fassung, deren Fortsatz sich durch den verengten Abschnitt erstreckt. Die Schulter kann eine Ringschulter sein. Insbesondere kann die Schulter eine ebene Ringfläche oder eine Ringfläche aufweisen, die auf einem Kegelmantel mit einem Öffnungswinkel größer als 90°, vorzugsweise größer als 120° oder 160° liegt. Die Ringfläche bildet eine Anschlagfläche für die Fassung. Vorzugsweise ist der Fortsatz mindestens so lang wie der verengte Abschnitt, sodass das Ende des Fortsatzes in Ausschubposition mit der stirnseitigen distalen Endfläche des Endstücks bündig steht oder über dieses hinaus ragt. Die Sitzöffnung für die Elektrode erstreckt sich vorzugsweise durch den gesamten Fortsatz in die Fassung hinein.

Weitere Einzelheiten vorteilhafter Ausführungsformen der Erfindung sind Gegenstand von Ansprüchen, und gehen zudem aus der Zeichnung und/oder der zugehörigen Beschreibung hervor. Es zeigen:
Figur 1 das erfindungsgemäße Instrument angeschlossen an ein speisendes Gerät, in schematisierter Darstellung,
Figur 2 das distale Ende des Instruments nach Figur 12, in vergrößerter perspektivischer Darstellung,
Figur 3 das distale Ende des Instruments nach Figur 1 und 2, in längsgeschnittener Prinzipdarstellung mit der Elektrode in Arbeitsposition (Ausschubposition),
Figur 4 das distale Element des Instruments entsprechend Figur 3, jedoch mit Fassung und Elektrode, in Rückzugsposition,
Figur 5 eine ausschnittsweise Darstellung des distalen Endes des Instruments, in längsgeschnittener vergrößerter Darstellung und
Figur 6 eine abgewandelte Ausführungsform des erfindungsgemäßen Instruments, in einer Darstellung entsprechend Figur 5.

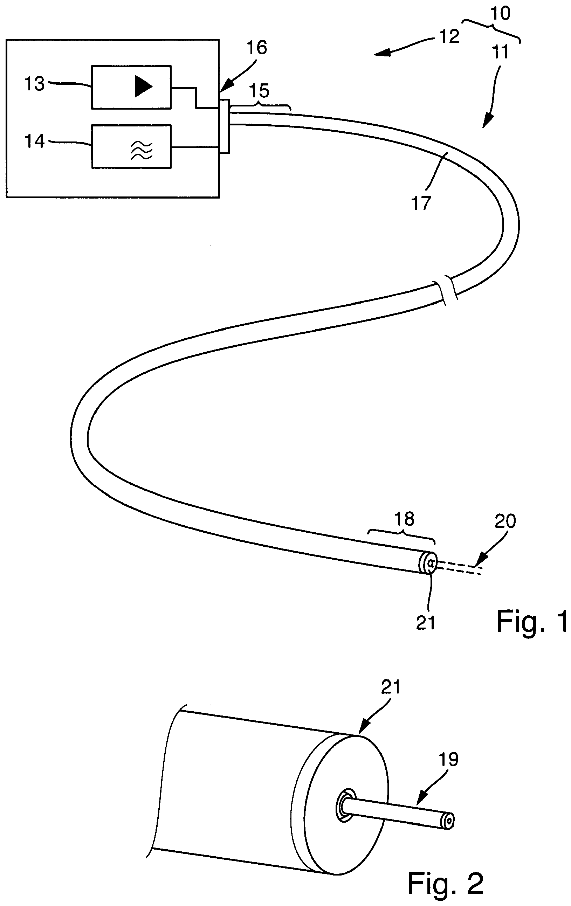

In Figur 1 ist eine chirurgische Einrichtung 10 veranschaulicht, zu der ein Instrument 11 sowie ein zur Speisung des Instruments 11 vorgesehenes Gerät 12 gehören. Das Gerät 12 ist dazu eingerichtet, unter Druck stehendes Fluid, insbesondere eine unter Druck stehende Flüssigkeit, wie beispielsweise Natriumchloridlösung, bereitzustellen und weist dazu eine entsprechende Speiseeinrichtung 13 auf. Außerdem enthält das Gerät 12 einen chirurgischen Generator 14, der zum Beispiel zur Abgabe von hochfrequenter Wechselspannung eingerichtet sein kann. Das Instrument 11 ist mit seinem proximalen Ende 15 an eine entsprechende Anschlusseinrichtung 16 des Geräts 12 angeschlossen und wird somit mit den bereitgestellten Medien, beispielsweise Flüssigkeit und Behandlungsstrom, versorgt. Der Generator 14 weist außerdem einen nicht veranschaulichten Neutralanschluss auf, an den eine ebenfalls nicht dargestellte Neutralelektrode anschließbar ist, die an einem Patienten angebracht werden kann.

Das Instrument 11 umfasst einen flexiblen Schlauch 17, der sich von dem proximalen Endbereich des Instruments 11 bis zu dessen distalen Endbereich 18 erstreckt. Ein solches Instrument kann beispielsweise endoskopisch eingesetzt werden, indem es durch den Arbeitskanal eines Endoskops in den Körper eines Patienten eingeführt wird, um dort chirurgische Behandlungen auszuführen. Das Instrument 11 ist dabei vorzugsweise ein Hybridinstrument, das sowohl die Behandlung von Gewebe mittels einer beispielsweise in Figur 2 veranschaulichten stift- oder nadelförmigen Elektrode 19 als auch die Behandlung mittels eines in Figur 1 schematisch angedeuteten Fluidstrahls 20 gestattet, mit dessen Hilfe Flüssigkeit in Gewebe eingebracht werden kann. So können beispielsweise in biologischem Gewebe Flüssigkeitsdepots angelegt werden, um eine Gewebeschicht von einer anderen Gewebeschicht zu trennen. Ein Anwendungsfall ist die Resektion von Gewebepartien in einem Hohlorgan, beispielsweise bei der Tumorentfernung, bei der unter dem Tumor zunächst ein Flüssigkeitskissen erzeugt und der Tumor dann mittels der Elektrode 19 herausgeschnitten wird. Das Flüssigkeitskissen trennt den Tumor dann von darunterliegendem Gewebe, um eine schonende Behandlung und Entfernung des Tumors zu ermöglichen.

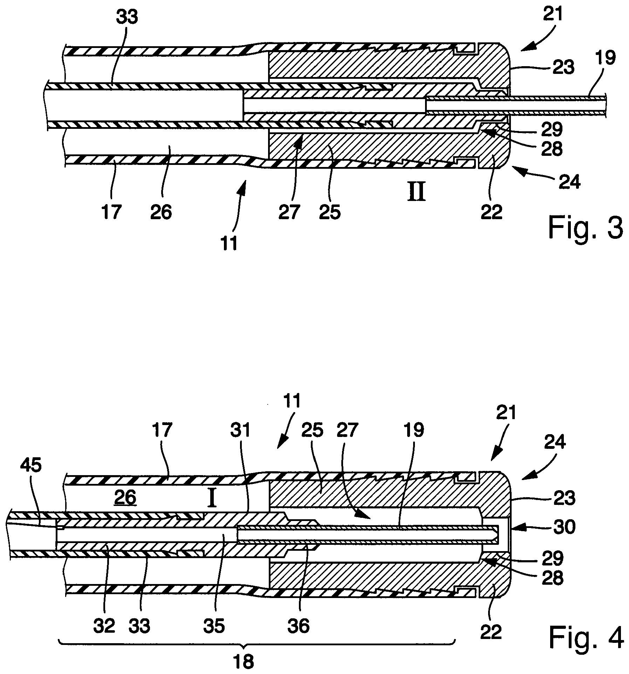

Das Instrument 11 weist an seinem distalen Endbereich 18 ein Endstück 21 auf, dessen Aufbau und Gestaltung sich insbesondere aus den Figuren 3 bis 6 ergibt. Das distale Endstück 21 ist beispielsweise aus Keramik oder einem anderen, vorzugsweise einem nichtleitenden Material, wie beispielsweise einem Kunststoff oder dergleichen ausgebildet. Das Endstück 21 weist einen Kopf 22 auf, dessen stirnseitige Endfläche 23 zugleich die distale Endfläche des Instruments 11 bildet. Der Kopf 22 ist, wie aus Figur 2 hervorgeht, etwa scheibenförmig ausgebildet, vorzugsweise mit einem gerundeten Übergang 24 von der distalen Endfläche 23 zur Umfangsfläche. An den Kopf 22 schließt sich ein Schaft 25 an, der in das Lumen 26 des Schlauchs 17 eingeschoben ist und an dem Kopf 22 anliegen kann. Das Endstück 21 ist vorzugsweise bezüglich der Längsachse des Schlauchs 17 rotationssymmetrisch ausgebildet und weist einen zentralen Durchgangskanal 27 auf. Dieser erstreckt sich durch den Schaft 25, der somit einen Rohrfortsatz bildet. Der Durchgangskanal 27 ist vorzugsweise etwa zylindrisch ausgebildet. An einer Ringschulter 28 verengt sich der Durchgangskanal 27 und geht dann in einen durchmesserreduzierten Abschnitt 29 über, der an der Endfläche 23 eine Mündungsöffnung 30 festlegt (Figur 4).

Anstelle des flexiblen Schlauchs kann auch ein starrer Schaft vorgesehen sein. Das Endstück 21 kann in den Schaft eingesetzt oder Teil des Schafts sein. Es kann auch einstückiger Bestandteil des Schlauchs sein.

In dem in den Figuren 3 und 4 dargestellten distalen Bereich 18 des Instruments 11 ist eine Fassung 31 für die Elektrode 19 angeordnet. Die Fassung 31 ist in dem Durchgangskanal 27 angeordnet und in diesem längsbeweglich gelagert. Von der Fassung 31 erstreckt sich ein Schlauchanschlussfortsatz 32 in proximaler Richtung. Auf dem Schlauchanschlussfortsatz 32 ist ein Fluidschlauch 33 gehalten und so mit der Fassung 31 verbunden. Der Fluidschlauch 33 erstreckt sich durch die gesamte Länge des Instruments bis zu seinem proximalen Ende. Der Fluidschlauch ist vorzugsweise zug- und drucksteif ausgebildet, um die Fassung 31 und die Elektrode 19 von dem proximalen Ende des Instruments her gezielt zwischen der Rückzugsposition und der Ausschubposition bewegen zu können.

Zur Versorgung der Elektrode 19 mit Behandlungsstrom kann die Fassung 31 über einen durch das Lumen des Fluidschlauchs 33 geführten Draht 45 bestromt werden. Der Draht 45 ist über die Anschlusseinrichtung 16 mit den chirurgischen Generator 14 verbunden. Alternativ kann der Draht 45 in der Wandung des Fluidschlauchs 33 angeordnet sein.

Die entsprechend spannungsbeaufschlagte und stromversorgte Elektrode 19 kann zum Schneiden von Gewebe eingesetzt werden, indem sie in das Gewebe eingestochen und dann seitlich bewegt wird. Die an der Elektrode 19 anliegende Spannung erzeugt Funken zwischen dem Gewebe und der Elektrode 19, die das Gewebe koagulieren und verdampfen. So kann ein Schnitt erzeugt werden.

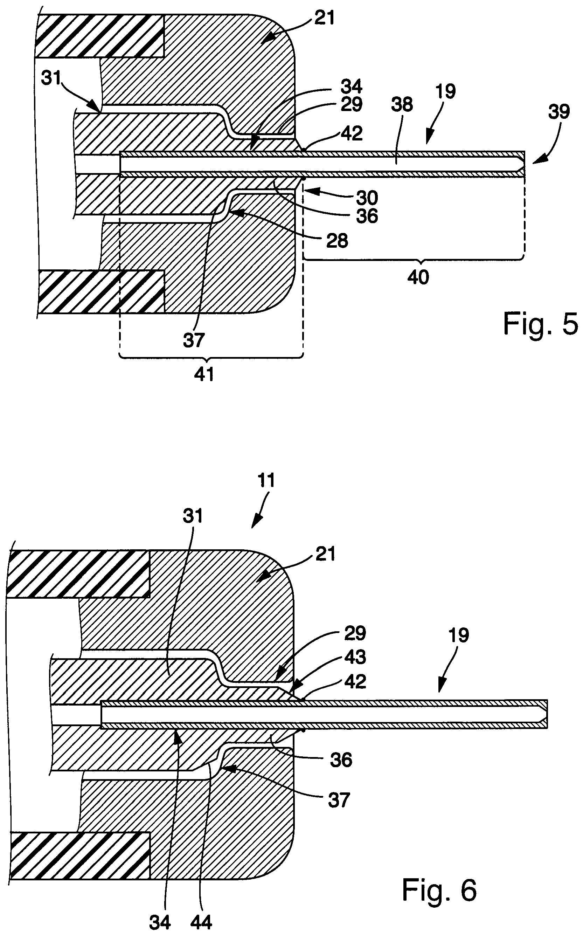

Die Fassung 31 weist, wie insbesondere auch aus Figur 5 hervorgeht, eine Sitzöffnung 34 auf, die sich koaxial zu der Fassung und dem Endstück 21 von dem distalen Ende her in die Fassung 31 hinein erstreckt. Die Sitzöffnung kommuniziert mit einem Fluidkanal 35, der sich bis zu dem proximalen Ende der Fassung 31 durch den Schlauchanschlussfortsatz 32 hindurch erstreckt und somit über den Fluidschlauch 33 mit Flüssigkeit versorgt werden kann.

Die Fassung 31 weist, wie aus Figur 5 hervorgeht, einen zylindrischen Abschnitt auf, der mit geringem Spiel axial beweglich in dem Durchgangskanal 27 sitzt. Von diesem zylindrischen Abschnitt erstreckt sich ein Fortsatz 36 in distaler Richtung durch den durchmesserreduzierten Abschnitt 29 des Endstücks 21 hindurch. Der Fortsatz 36 endet dabei an der Mündungsöffnung 30 oder steht, wie Figur 5 andeutet, geringfügig über diese hinaus.

Zwischen dem Fortsatz 36 und dem übrigen Abschnitt der Fassung 31 ist eine Schulter 37 gebildet, die mit der Ringschulter 28 in Anlage kommen kann. Liegt die Schulter 37 an der Ringschulter 28 an, steht die Fassung 31 und mit ihr die Elektrode 19 in Ausschubposition gemäß Figur 3. Ist die Fassung 31 jedoch in proximaler Richtung verlagert, sodass die Elektrode 19 an oder in proximaler Richtung hinter der distalen Endfläche 23 steht, befindet sich die Fassung 31 und mit ihr die Elektrode 19 in Rückzugsposition. Die Position der Fassung 31 und der Elektrode 19 kann über den Fluidschlauch 33 gesteuert werden, indem dieser in distaler oder proximaler Richtung axial verlagert wird. Entsprechende Mittel zur axialen Bewegung des Fluidschlauchs 23 können an dem proximalen Ende 15 oder an andrer Stelle vorgesehen sein und sind in den Figuren nicht weiter veranschaulicht.

Die Elektrode 19 kann als Kapillarrohr, d.h. als dünnwandiger, schlanker Hohlzylinder ausgebildet sein, der einen Fluidkanal 38 umschließt. Der Fluidkanal kann an dem distalen Ende der Elektrode 19 in einer Düsenöffnung 39 enden. Die Düsenöffnung 39 kann durch das offene Ende des Fluidkanals 38 gebildet sein. Alternativ kann die Düsenöffnung 39, wie in Figur 5 schematisch angedeutet, durch eine Verengung des Fluidkanals 38 an seinem distalen Ende gebildet sein.

Die Elektrode 19 kann beispielsweise aus Hartmetall, Wolfram oder dergleichen bestehen. Sie weist einen aus der Fassung 31 herausragenden Abschnitt 40 und einen in der Sitzöffnung 34 gehaltenen Abschnitt 41 auf. Der Abschnitt 41 ist dabei vorzugsweise länger als der Fortsatz 36 und somit auch länger als der durchmesserreduzierte Abschnitt 29. Die Länge des Abschnitts 40 ist vorzugsweise mehrfach größer als der Durchmesser der Elektrode 19. Insbesondere ist die Länge 40 vorzugsweise mehr als 5 mal, weiter vorzugsweise mehr als 10 mal so groß, wie der Durchmesser des Abschnitts 40.

Die Elektrode 19 ist mit der Fassung 31 vorzugsweise stoffschlüssig verbunden. Dazu kann der Fortsatz 36 insbesondere an seinem stirnseitigen Ende mit der Elektrode 19 über eine oder mehrere Schweißverbindungen 42 verbunden sein. Die Schweißverbindung 42 kann beispielsweise eine Laserschweißverbindung sein. Die Laserschweißverbindung kann eine Schweißnaht oder einen oder mehrere Schweißpunkte umfassen.

Figur 6 veranschaulicht das oben beschriebene Instrument 11 mit der nachfolgend beschriebenen Abwandlung:
Der Fortsatz 36 weist an seinem distalen Ende eine konische Verjüngung 43 (d.h. einen konischen Abschnitt 43) auf, die an der Schweißverbindung 42 endet. Die konische Verjüngung 43 kann als Einführhilfe dienen, wenn die Fassung 31 aus ihrer Rückzugsposition in die Ausschubposition fährt und dabei der Fortsatz 36 in den durchmesserreduzierten Abschnitt des Endstücks 21 eindringen muss. Außerdem kann der konische Abschnitt 43 dazu genutzt werden, lokale Spannungen in der Elektrode 19 in der Umgebung der Schweißverbindung 42 zu reduzieren, wenn die Elektrode 19 mit Radialkräfte belastet wird.

Außerdem kann die Schulter 37 einen konischen äußeren Bereich 44 aufweisen. Dieser dient als Einführhilfe beim Einführen der Fassung 31 in das Endstück 21, was die Montage erleichtert.

Im Übrigen gelten ergänzend die bereits zu den Figuren 1 bis 5 gegebene Erläuterungen und Erklärungen hinsichtlich Struktur und Funktion.

Die insoweit beschriebene chirurgische Einrichtung 10 arbeitet wie folgt:
In Betrieb ist das Instrument 11 an das Gerät 12 angeschlossen. Die jeweilige Betriebsart, nämlich Unterspritzen oder Resektion, wählt der Nutzer über nicht weiter veranschaulichte Steuermittel, beispielsweise ein Pedal. Das Instrument 11 ist durch eine geeignete Vorrichtung, beispielsweise ein Endoskop, in den Körper des Patienten eingeführt. Will der Operateur nun beispielsweise eine Gewebepartie unterspritzen, überführt er die Fassung 31 und mit ihr die Elektrode 19 in Rückzugsposition gemäß Figur 4. Durch Aktivierung der Fluidquelle 13 kann er über den Fluidschlauch 33 Fluid in den Fluidkanal 38 der hohlen Elektrode 19 fördern, sodass dieser an der Düsenöffnung 39 als scharfer Strahl 20 austritt. Zum Unterspritzen kann der Behandler die distale Endfläche 23 des Instruments 11 auf das betreffende Körpergewebe aufsetzen. Der scharfe Fluidstrahl 20 dringt in das Gewebe ein und erzeugt ein Flüssigkeitsdepot darin.

Nachdem dieser Vorgang beendet ist, kann der Behandler das Gerät 12 und das Instrument 11 vom Unterspritzungsmodus in den Resektionsmodus überführen. Dazu wird die Fassung 31 und mit ihr die Elektrode 19 in die Ausschubposition gemäß Figur 3, 5 oder 6 überführt. Die Fassung 31 wird dabei soweit in distaler Richtung bewegt, dass ihre Schulter 37 an der Ringschulter 28 ihre Anlage findet. Die Speiseeinrichtung13 wird deaktiviert und der Generator 14 aktiviert.

Mit der entsprechend spannungsbeaufschlagten und stromversorgten Elektrode 19 kann nun wie mit einem Skalpell ein gewünschter Gewebebereich aus dem Gewebezusammenhang herausgeschnitten und danach aus dem Körper des Patienten entfernt werden.

Bei der Anwendung des Instruments 11 kann beliebig oft zwischen dem Unterspritzungsmodus und dem Resektionsmodus gewechselt werden.

Das erfindungsgemäße elektrochirurgische Instrument 11 weist einen als Schlauch 17 oder einen als hohler Stab ausgebildeten Grundkörper auf, an dem ein Endstück 21 ausgebildet oder in den ein Endstück 21 eingesetzt ist. Das Endstück 21 weist einen Durchgangskanal auf 27 auf, der an seinem distalen Ende einen verengten Abschnitt 29 aufweist. Die Elektrode 19 ist in einer Fassung 31 gehalten, die einen Fortsatz 36 aufweist, der die Elektrode 19 umgibt. Der Fortsatz 36 ist darauf eingerichtet, sich durch den verengten Abschnitt 29 des Durchgangskanals 27 bis zu der Endfläche 23 zu erstrecken, sodass er an der Mündungsöffnung 30 des verengten Abschnitts 29 zu Tage tritt, wenn die Elektrode 19 in Ausschubposition steht. An diesem an der Mündungsöffnung 30 stehenden Abschnitt des Fortsatzes 36 ist die Elektrode 19 mit dem Fortsatz 36 verbunden, vorzugsweise verschweißt.

### Bezugszeichen:

- 10: chirurgische Einrichtung
- 11: Instrument
- 12: Gerät
- 13: Fluidquelle
- 14: chirurgischer Generator
- 15: proximaler Endbereich des Instruments 11
- 16: Anschlusseinrichtung
- 17: Schlauch
- 18: distaler Endbereich des Schlauchs
- 19: Elektrode
- 20: Fluidstrahl
- 21: Endstück
- 22: Kopf
- 23: distale Endfläche des Instruments 11
- 24: gerundeter Übergang
- 25: Schaft
- 26: Lumen des Schlauchs 17
- 27: Durchgangskanal des Endstücks 21
- 28: Ringschulter
- 29: durchmesserreduzierter Abschnitt des Durchgangskanals 27
- 30: Mündungsöffnung
- 31: Fassung
- I: Rückzugsposition der Fassung 31
- II: Ausschubposition der Fassung 31
- 32: Schlauchanschlussfortsatz
- 33: Fluidschlauch
- 34: Sitzöffnung
- 35: Fluidkanal der Fassung 31
- 36: Fortsatz
- 37: Schulter
- 38: Fluidkanal der Elektrode 19
- 39: Düsenöffnung
- 40: distaler freitragender Abschnitt der Elektrode 19
- 41: in der Sitzöffnung 34 gehaltener Abschnitt der Elektrode 19
- 42: Schweißverbindung
- 43: konische Verjüngung des Fortsatzes 36
- 44: konischer Bereich der Schulter 37
- 45: Draht

## Patentansprüche

1. Elektrochirurgisches Instrument (11), insbesondere Hybridinstrument mit Fluidejektionsfunktion,
mit einem distalen Endstück (21), das eine distale Endfläche (23) und einen Durchgangskanal (27) aufweist, wobei die distale Endfläche (23) eine Mündungsöffnung (30) umschließt, an der der Durchgangskanal (27) mündet,
mit einer Fassung (31), die in dem Durchgangskanal (27) zwischen einer Rückzugsposition (I), in der die Fassung von der Endfläche (23) entfernt steht, und einer Ausschubposition (II), in der die Fassung (31) mit der Endfläche (23) bündig abschließend oder aus dieser heraus ragend steht, linear beweglich angeordnet ist und die eine Sitzöffnung (34) aufweist,
mit einer Elektrode (19), die mit einem Abschnitt (41) in der Sitzöffnung (34) angeordnet und mit einem anderen Abschnitt (40) aus dieser heraus ragend gehalten ist.

2. Instrument nach Anspruch 1, wobei die distale Endfläche (23) den distalen Abschluss des Instruments (11) bildend angeordnet ist, der nur von der Elektrode (19) in Distalrichtung überragt ist, wenn die Elektrode (19) in der Ausschubposition (II) steht.

3. Instrument nach Anspruch 1 oder 2, wobei das distale Endstück (21) einen Kopf (22) aufweist, an dem die distale Endfläche (23) ausgebildet ist, wobei sich von dem Kopf (22) ein Schaft (25) weg erstreckt, auf dem ein Schlauch (17) aufgenommen ist, wobei der Kopf (22) einen Kopfdurchmesser und der Schaft (25) einen Schaftdurchmesser aufweist, der geringer als der Kopfdurchmesser ist.

4. Instrument nach einem der vorstehenden Ansprüche, wobei der Durchgangskanal (27) einen zylindrischen Abschnitt aufweist, an den sich in distaler Richtung ein verengter Abschnitt (29) anschließt, der an der Mündungsöffnung (30) endet.

5. Instrument nach Anspruch 4, wobei zwischen dem zylindrischen Abschnitt und dem verengten Abschnitt (29) eine Ringschulter (28) ausgebildet ist.

6. Instrument nach einem der Ansprüche 4 oder 5, wobei der verengte Abschnitt (29) zylindrisch ausgebildet ist.

7. Instrument nach einem der Ansprüche 3 bis 6, wobei die Fassung (31) einen Fortsatz (36) aufweist, der sich durch den Kopf (22) erstreckt.

8. Instrument nach Anspruch 7, wobei der Fortsatz (36) mindestens so lang ist, wie der verengte Abschnitt (29).

9. Instrument nach Anspruch 7 oder 8, wobei sich die Sitzöffnung (34) durch den Fortsatz (36) hindurch erstreckt.

10. Instrument nach einem der vorstehenden Ansprüche, wobei die Elektrode (19) zylindrisch ausgebildet ist.

11. Instrument nach einem der vorstehenden Ansprüche, wobei die Elektrode (19) mit der Fassung (31) verschweißt ist.

12. Instrument nach einem der vorstehenden Ansprüche, wobei der aus der Sitzöffnung (34) heraus ragende Abschnitt (40) der Elektrode (19) eine Länge aufweist, die das Fünffache seines Durchmessers übersteigt.

13. Instrument nach einem der vorstehenden Ansprüche, wobei die Elektrode (19) einen Fluidkanal (38) aufweist.

14. Instrument nach einem der vorstehenden Ansprüche, wobei die Fassung (34) mit einem Fluidschlauch (33) verbunden ist.

15. Instrument den Ansprüchen 3 und 14, wobei der Fluidschlauch (33) sich durch den Schlauch (17) erstreckend angeordnet ist.

## Claims

1. Electrosurgical instrument (11), particularly hybrid instrument with fluid injection function,
having a distal end piece (21) that comprises a distal end surface (23) and a passage channel (27), whereby the distal end surface (23) surrounds a mouth opening (30) at which the passage channel (27) opens out,
having a holder (31) that is linearly movably arranged in the passage channel (27) between a retracted position (I) in which the holder is arranged distant from the end surface (23) and an extended position (II) in which the holder (31) ends flush with the end surface (23) or projects beyond it, wherein the holder (31) comprises a seat opening (34),
having an electrode (19) that is arranged with one section (41) in the seat opening (34) and is held to project therefrom with another section (40).

2. Instrument according to claim 1, wherein the distal end surface (23) is arranged forming the distal end of instrument (11) beyond which only the electrode (19) projects in distal direction, if the electrode (19) is in the extended position (II).

3. Instrument according to claim 1 or 2, wherein the distal end piece (21) comprises a head (22) on which the distal end surface (23) is formed, wherein a shank (25) extends from the head (22) on which a hose (17) is held, wherein the head (22) comprises a head diameter and the shank (25) comprises a shank diameter that is less than the head diameter.

4. Instrument according to any of the preceding claims, wherein the passage channel (27) comprises a cylindrical section to which a narrowed section (29) adjoins in distal direction that ends at the mouth opening (30).

5. Instrument according to claim 4, wherein a ring shoulder (28) is formed between the cylindrical section and the narrowed section (29).

6. Instrument according to one of the claims 4 or 5, wherein the narrowed section (29) is configured cylindrically.

7. Instrument according to one of the claims 3 to 6, wherein the holder (31) comprises an extension (36) that extends through the head (22).

8. Instrument according to claim 7, wherein the extension (36) is at least as long as the narrowed section (29).

9. Instrument according to claim 7 or 8, wherein the seat opening (34) extends through the extension (36).

10. Instrument according to any of the preceding claims, wherein the electrode (19) is configured cylindrically.

11. Instrument according to any of the preceding claims, wherein the electrode (19) is welded to the holder (31).

12. Instrument according to any of the preceding claims, wherein the section (40) of the electrode (19) projecting from the seat opening (34) has a length that exceeds the fivefold of its diameter.

13. Instrument according to any of the preceding claims, wherein the electrode (19) comprises a fluid channel (38) .

14. Instrument according to any of the preceding claims, wherein the holder (31) is connected with a fluid hose (33) .

15. Instrument according to claims 3 and 14, wherein the fluid hose (33) is arranged in a manner extending through the hose (17).

## Revendications

1. Instrument électrochirurgical (11), en particulier instrument hybride à fonction d'éjection de fluide,
comprenant une pièce d'extrémité distale (21) qui présente une surface d'extrémité distale (23) et un conduit de passage (27), la surface d'extrémité (23) distale entourant un orifice d'embouchure (30) dans lequel débouche le conduit de passage (27),
comprenant un châssis-support (31) qui est disposé dans le conduit de passage (27) avec possibilité de déplacement linéaire entre une position de retrait (I), dans laquelle le châssis-support se trouve à distance de la surface d'extrémité (23), et une position de déploiement (II) dans laquelle le châssis-support (31) est affleurant avec la surface d'extrémité (23) ou dépasse de celle-ci, et qui présente une ouverture formant siège (34),
comprenant une électrode (19) qui est disposée avec une portion (41) dans l'ouverture formant siège (34) et est tenue de manière à dépasser de celle-ci avec une autre portion (40).

2. Instrument selon la revendication 1, dans lequel la surface d'extrémité (23) distale est disposée de manière à former la fermeture distale de l'instrument (11), par rapport à laquelle seule l'électrode (19) dépasse dans la direction distale, lorsque l'électrode (19) occupe la position de déploiement (II).

3. Instrument selon la revendication 1 ou 2, dans lequel la pièce d'extrémité (21) distale présente une tête (22) sur laquelle est réalisée la surface d'extrémité distale (23), un fût (25) s'étendant à partir de la tête (22), fût sur lequel est rapporté un tube (17), la tête (22) présentant un diamètre de tête, et le fût (25) un diamètre de fût qui est inférieur au diamètre de tête.

4. Instrument selon l'une des revendications précédentes, dans lequel le conduit de passage (27) présente une portion cylindrique à laquelle se raccorde, dans la direction distale, une portion rétrécie (29) qui se termine à l'orifice d'embouchure (30).

5. Instrument selon la revendication 4, dans lequel un épaulement annulaire (28) est réalisé entre la portion cylindrique et la portion rétrécie (29).

6. Instrument selon l'une des revendications 4 ou 5, dans lequel la portion rétrécie (29) est réalisée sous une forme cylindrique.

7. Instrument selon l'une des revendications 3 à 6, dans lequel le châssis-support (31) présente un prolongement (36) qui s'étend à travers la tête (22).

8. Instrument selon la revendication 7, dans lequel le prolongement (36) présente une longueur qui est au moins égale à celle de la portion rétrécie (29).

9. Instrument selon la revendication 7 ou 8, dans lequel l'ouverture formant siège (34) s'étend à travers le prolongement (36).

10. Instrument selon l'une des revendications précédentes, dans lequel l'électrode (19) est réalisée sous une forme cylindrique.

11. Instrument selon l'une des revendications précédentes, dans lequel l'électrode (19) est soudée au châssis-support (31).

12. Instrument selon l'une des revendications précédentes, dans lequel la portion (40) de l'électrode (19) qui dépasse de l'ouverture formant siège (34) présente une longueur qui est supérieure à cinq fois son diamètre.

13. Instrument selon l'une des revendications précédentes, dans lequel l'électrode (19) présente un canal de fluide (38).

14. Instrument selon l'une des revendications précédentes, dans lequel le châssis-support (34) est raccordé à un tube de fluide (33).

15. Instrument selon les revendications 3 et 14, dans lequel le tube de fluide (33) est disposé de manière à s'étendre dans le tube (17).
